# EUROPEAN PATENT SPECIFICATION

(11) **EP 2 100 547 B1**
(45) Date of publication and mention of the grant of the patent: **12.02.2014**
(21) Application number: 09003394.5
(22) Date of filing: 09.03.2009
(51) Int. Cl.: A61B 1/05, A61B 1/005

(54) **Endoscope insertion portion**
Endoskopeinführteil
Portion d'insertion d'endoscope

(30) Priority: 10.03.2008 JP 2008059118
(43) Date of publication of application: 16.09.2009
(73) Proprietor: Fujifilm Corporation, Tokyo (JP)
(72) Inventor: YAMAZAKI, Masayuki, Saitama (JP)
(74) Representative: Klunker . Schmitt-Nilson . Hirsch

(56) References cited:
- EP-A- 0 524 755
- DE-A1-102004 010 193
- US-A- 5 174 277
- US-A1- 2002 032 371
- US-A1- 2002 058 858
- US-B1- 6 482 149

## Description

### BACKGROUND OF THE INVENTION

### 1. Field of the Invention

The present invention relates to an endoscope insertion portion used for medical purposes and the like and particularly to a structure of a bending tube portion thereof.

### 2. Description of the Related Art

In an endoscope, a base end portion of an insertion portion to be inserted into a body cavity is coupled to a main body operation portion, and the insertion portion is constituted by a distal-end rigid portion, a bending tube portion, and a flexible tube portion from the distal end side. At the distal-end rigid portion, endoscope observing means provided at least with an illumination portion and an observation portion is provided, and in order to orient the distal-end rigid portion in a desired direction, the bending tube portion is made capable of being bent by remote control from the main body operation portion. The bending tube portion is made in a node-ring structure having a predetermined length by sequentially and pivotally attaching bending rings formed in a ring shape.

A bending device of the endoscope bends a bending portion in an intended direction by a pushing / pulling operation of one or a plurality of operation wires inserted through the bending tube portion. For this bending operation, an operation knob for manual operation provided at the main body operation portion and a pulley rotationally moved by an operation of the operation knob are provided, and the operation wire is coupled to and provided at the pulley. The operation wire is led out of the pulley and has the distal end thereof fixed to the bending ring at the most distal end constituting the bending tube portion or the distal-end rigid portion.

The flexible tube portion coupled to the bending tube portion has flexibility in the bending direction, and in order to prevent the operation wire from being pulled out when the flexible tube portion is bent, the operation wire is inserted through a flexible sleeve made up of a contact coil and the like in the flexible tube portion so as to give some extra length to the operation wire. On the other hand, in the bending tube portion, in order not to lower follow-up performance of the bending tube portion during operation of the operation knob and in order to bend the bending tube portion in an intended direction without fail, the operation wire is provided having the distal end portion fixed to the bending ring at the most distal end or the distal-end rigid portion. Also, in the flexible tube portion, the operation wire is led out of the flexible sleeve, and in the bending tube portion, a guide mechanism so as not to cause displacement in the circumferential direction is provided.

The guide mechanism of the operation wire in the bending tube portion uses a method disclosed in Japanese Unexamined Patent Application Publication No. 2002-78674, for example. In this method, a guide hole for the guide mechanism of the operation wire is provided at a caulking pin coupling a coupling portion of the bending ring constituting the bending tube portion. In Japanese Unexamined Patent Application Publication No. 2002-78674, contact of the operation wire with the coupling portion is prevented by forming a notch portion in the coupling portion of the bending ring, by which damage to the operation wire is prevented. An example of the guide mechanism in another method is illustrated in Fig. 1 of Japanese Unexamined Patent Application Publication No. 2005-58571. That is, a cut and drawn portion through which the operation wire is inserted is provided at each bending ring constituting the bending tube portion, and the cut and drawn portion is used as a guide mechanism of the operation wire.

EP 0 524 755 discloses a flexible endoscope with a bending mechanism.

### SUMMARY OF THE INVENTION

The bending tube portion is provided for orienting the distal-end rigid portion in a desired direction, and the node-ring structure of the bending ring is constituted by a predetermined number of bending rings sequentially coupled by a pivot pin. The bending ring is in a shape diagonally cut open toward a direction away from the coupling portion (portion coupled by the pivot pin) so that the space between the bending ring of the front side and the bending ring of the rear side progressively widens, and a predetermined interval is formed between the front and rear bending rings. Furthermore, the rings can be displaced so as to be close to / away from each other around the pivot pin by a portion corresponding to the predetermined interval, by which the bending tube portion can be bent in an arc state, and an observation field of view of the distal-end rigid portion can be oriented in a desired direction.

When the bending tube portion is bent, a tension acts on the operation wire on the inner circumference side among the operation wires inserted through the bending tube portion, and the wire is made to slide while being pressed onto the inner circumferential face of the bending ring. Here, the bending ring is in the cut-open state having edges formed at the both ends, and if the pushing / pulling operation is conducted on the operation wire in this state, the operation wire on the inner circumference side is brought into contact with and rubbed by the edges. Then, the operation wire on the inner circumference side might be damaged. In addition, the bending operation of the bending tube portion is conducted repeatedly in order to orient the observation field of view of the endoscope to a desired direction in the body cavity, abrasion caused by rubbing on the operation wire by the edge progresses and the operation wire might be cut off in the end, which is a problem in durability. Also, if the operation wire is rubbed by the edge, the pushing / pulling operation is conducted in a state under a frictional force, and there is another problem of lowered operational feeling.

Guide mechanisms for the operation wire mainly include the above-mentioned two methods. In the case of provision of a guide hole at the coupling portion of the bending ring, as disclosed in Japanese Unexamined Patent Application Publication No. 2002-78674, formation of a notch portion at the coupling portion is extremely effective in the point that the operation wire is not damaged. However, as shown in Fig. 1 of Japanese Unexamined Patent Application Publication No. 2002-78674, this method is intended for those with some interval provided between the guide hole formed at a caulking pin and the inner circumferential face of the bending ring. If an allowance is given to the interval, the operation wire inserted through the guide hole is not brought into contact with the inner circumferential face of the bending ring, but the operation wire protrudes inward by the same amount, which reduces a capacity for the contents and the number of contents that can be inserted through the bending tube portion. On the other hand, if ensuring of the number of contents is given priority, the diameter of the bending tube portion becomes large.

Then, in the case intended for insertion of the operation wire by providing a cut and drawn section at the bending ring, as mentioned above, there is a problem that the operation wire is damaged by the rubbing to the edge of the bending ring when the bending tube portion is bent. However, since there is substantially no interval provided between the operation wire and the bending ring inner circumferential face, no such problem of enlarged diameter of the endoscope insertion portion or decrease in the number of contents is caused.

Thus, the present invention as defined by claim 1 has an object to protect the operation wire and to improve operational feeling regardless of a bent angle of the bending tube portion.

An endoscope insertion portion in a first aspect of the present invention is an endoscope insertion portion provided with a distal-end rigid portion having at least an illumination portion and an observation portion, a bending tube portion coupled to the distal-end rigid portion and to which a plurality of bending rings are coupled by a pivot attachment portion, and a flexible tube portion coupled to the bending tube portion and a main body operation portion, **characterized in that** the bending ring is provided with an insertion path through which an operation wire for bending the bending tube portion by a remote control from the main body operation portion is inserted, and an inward projection portion is provided in the front and rear positions in the axial direction of the insertion path.

According to the endoscope insertion portion in the first aspect, even if the operation wire is pressed toward an inner circumferential face of the bending ring when the bending tube portion is bent, the operation wire is brought into contact with the projection portion and will no longer move toward an edge. Thus, the operation wire is not rubbed by the edge, the operation wire can be protected, and operability can be improved. From the viewpoint that the problem of rubbing between the operation wire and the edge is completely solved, the projection portion preferably has such a height that the operation wire and the edge do not contact each other, but even if the operation wire and the edge are in somewhat contact with each other, the pressing force of the operation wire toward the edge is relieved by the provision of the projection portion, and thus, the projection portion can be set slightly lower. In addition, since the projection portion is a portion in direct contact with the operation wire, the portion is preferably formed in a smooth shape, and a spot projecting the most inward is preferably formed flat.

The endoscope insertion portion in a second aspect of the present invention is **characterized in that,** in the endoscope insertion portion described in the first aspect, the projection portion is given an extra width in the circumferential direction of the bending ring.

According to the endoscope insertion portion in the second aspect, since the projection portion is given the extra width in the circumferential direction, even if the operation wire is displaced in the circumferential direction, the wire does not drop from the projection portion any longer. The insertion path regulates an operation of the operation wire in the circumferential direction, but if there is no gap between them, the operability of the operation wire is lowered, and some allowance is given to the gap. Thus, the operation wire might be slightly operated in the circumferential direction, but even in this case, the extra width given to the bending ring prevents removal from the projection portion.

The endoscope insertion portion described in a third aspect of the present invention is **characterized in that,** in the endoscope insertion portion in the first or second aspect, the projection portion is formed in a recess state.

According to the endoscope insertion portion in the third aspect, since the projection portion is formed in the recess state, a function to regulate the operation of the operation wire in contact with the projection portion in the circumferential direction can be given to the projection portion, and removal of the operation wire can be prevented. By forming the projection portion in a largely recessed state, the operation wire can be surely brought into contact with the projection portion, but a height difference for the recess is required, which leads to an enlarged diameter of the bending portion. Thus, the height difference is preferably set to the minimum that prevents the removal of the operation wire.

The endoscope insertion portion in a fourth aspect of the present invention is **characterized in that,** in the endoscope insertion portion described in any one of the first to third aspects, the height of the projection portion is set so that "H ≤ h - d" is satisfied when the height of the projection portion is H, the height of the insertion path is h, and the diameter of the operation wire is d.

According to the endoscope insertion portion in the fourth aspect, since the height of the projection portion does not have to be higher than necessary if the height H of the projection portion is set such that the equation is satisfied, the capacity of the flexible tube portion is not pressed, and damage to the operation wire can be avoided.

### Advantage of the Invention

The endoscope of the present invention can prevent the operation wire from being rubbed by the edge of the bending ring by providing the projection portion on the bending ring, protect the operation wire and improve the operating feeling.

### BRIEF DESCRIPTION OF THE DRAWINGS

The above and other objects, configurations, actions and effects of the invention will be made apparent from embodiments of the invention illustrated below. The present invention is not limited to the embodiment of the invention which will be described below.
Fig. 1 is an entire configuration diagram of an endoscope;
Fig. 2 is a front view of a distal-end rigid portion of an insertion portion in the endoscope in Fig. 1;
Fig. 3 is a configuration explanatory diagram illustrating a bending tube portion and the distal-end rigid portion in the insertion portion of the endoscope;
Fig. 4 is a sectional view illustrating a part of the bending tube portion;
Fig. 5 is a configuration explanatory diagram similar to Fig. 3 when the bending tube portion is in the maximum bent state;
Fig. 6 is a sectional view similar to Fig. 4 when the bending tube portion is bent to some degree;
Fig. 7 is a sectional view similar to Fig. 4 when the bending tube portion is in the maximum bent state; and
Fig. 8 is a sectional view illustrating a projection portion and a cut and drawn portion of the bending tube portion.

### DESCRIPTION OF THE PREFERRED EMBODIMENTS

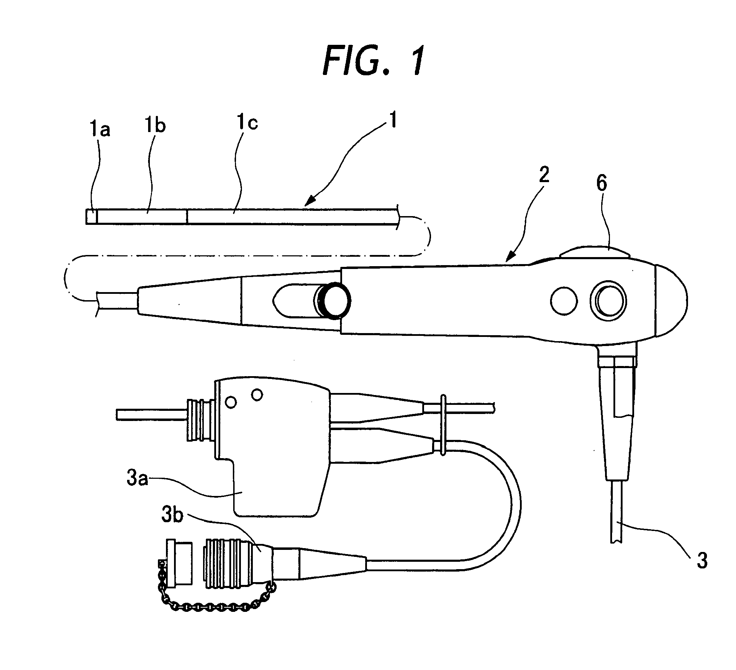

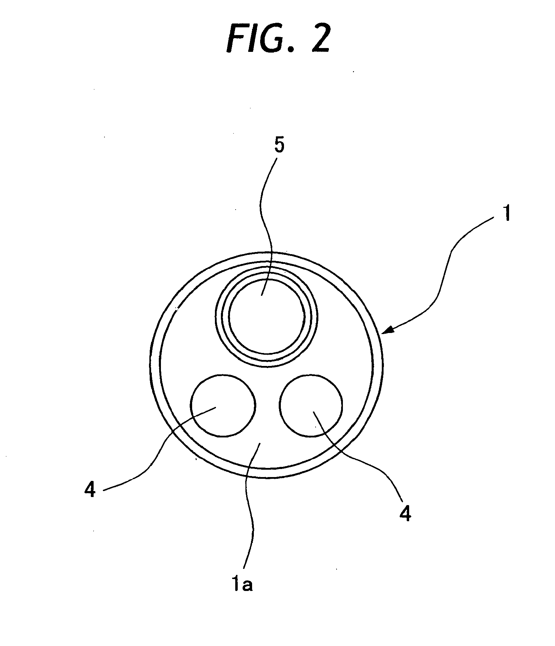

The present invention will be described below referring to the attached drawings. In Fig. 1, an endoscope of the present invention substantially comprises an insertion portion 1, a main body operation portion 2, and a universal cord 3, and the insertion portion 1 is provided with a distal-end rigid portion 1a, a bending tube portion 1b, and a flexible tube portion 1c. A base end portion of the flexible tube portion 1c is coupled to the main body operation portion 2, and this main body operation portion 2 is provided to be grasped by an operator operating the endoscope by hand for operation. The universal cord 3 extends from the main body operation portion 2 and is provided with connectors 3a, 3b to be detachably connected to a light-source device and a processor, not shown. At a distal end face of the distal-end rigid portion 1a in the insertion portion 1, as shown in Fig. 2, endoscope observing means provided with two illumination portions 4, 4 and an observation portion 5 is provided. Therefore, in a state where the insertion portion 1 is inserted into a body cavity, the inside of the body cavity is observed from the observation portion 5 under illumination of illumination light from the illumination portions 4, 4.

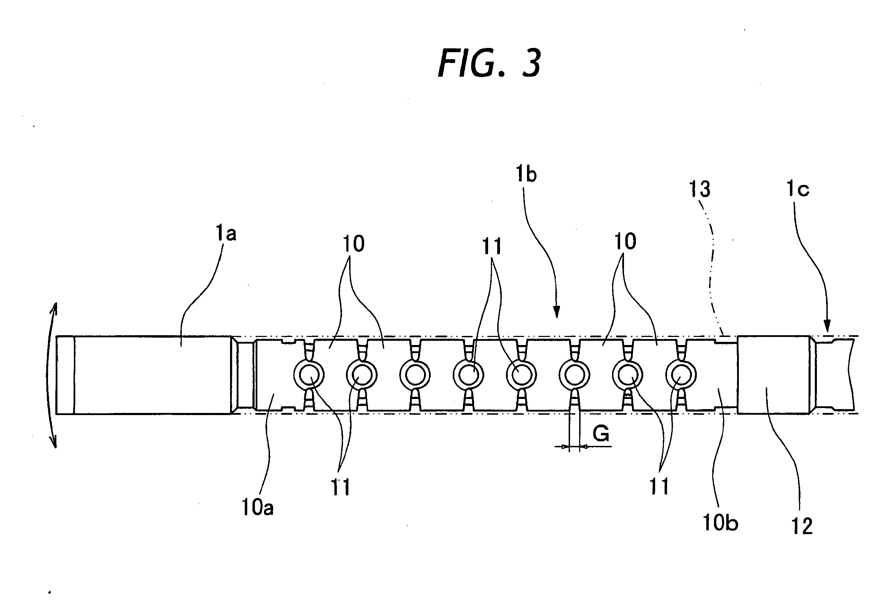

The bending tube portion 1b is provided in order to orient the distal-end rigid portion 1a in a desired direction. The bending tube portion 1b is, as shown in Fig. 3, constituted by predetermined bending rings 10 sequentially coupled by pivot pins 11. An interval G is formed between the front and rear bending rings 10, 10 so that they can be displaced close to / away from each other by a portion corresponding to the interval G around the pivot pin 11. Among the bending rings 10, the bending ring 10a at the most distal end is coupled to the distal-end rigid portion 1a, while the bending ring 10b at the most base end is coupled to a base 12, and the flexible tube portion 1c is coupled through the base 12. The bending tube portion 1b is bent in an arc shape so that the direction of the observation field of view by the endoscope observing means formed at the distal-end rigid portion 1a can be changed. Here, the bent direction of the bending tube portion 1b is a direction shown by an arrow in Fig. 3 and the portion is capable of being bent in two vertical directions. On an outer circumference portion of a coupling body of the bending rings 10 constituting a structural body of the bending tube portion 1b, a net and a sheath layer 13 as shown by a virtual line in Fig. 3 are provided.

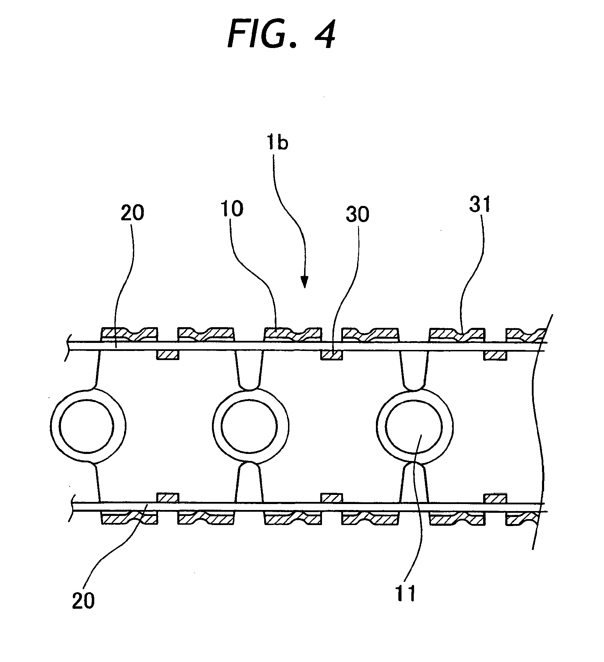

The bending tube portion 1b is capable of being bent as mentioned above, and in order to bend and operate the bending tube portion 1b, a bending operation knob 6 constituting a bending operation member is provided at the main body operation portion 2 as shown in Fig. 1. A rotary pulley, not shown, is provided at the bending operation knob 6, and a pair of operation wires 20, 20 as shown in Fig. 4 are connected to the pulley. Each of the operation wires 20 provided in the vertical direction are inserted through the flexible tube portion 1c, in which the distal end side is fixed to a cut and drawn portion formed at the bending ring at the most distal end of the bending tube portion 1b through soldering and the like so as to be folded back. The bending tube portion 1b is, as has already been described, capable of bending operation in the vertical direction, and the bending operation is carried out by operating the bending operation knob 6 provided at the main body operation portion 2. As the rotation of the pulley by means of the bending operation, one of the two inserted operation wires 20, 20 is pulled into the main body operation portion 2 side, while the other is fed out to the insertion portion 1 side. Therefore, the bending tube portion 1b of the insertion portion 1 is bent in a direction along which the operation wire 20 on the side pulled into the main body operation portion 2 is located.

Each of the bending rings 10 constituting the bending tube portion 1b has a substantially cylindrical shape and both ends thereof are formed in a shape diagonally cut open in directions so as to be vertically separated from the pivot pin 11 so as to allow bending in the vertical direction. In addition, a cut and drawn portion 30 is formed at each of the bending rings 10 as an insertion path, and an operation in the circumferential direction is regulated by inserting the operation wire 20 through the cut and drawn portion 30. That is, the cut and drawn portion 30 functions as positioning guide means for holding so as not to cause displacement. Therefore, the cut and drawn portion 30 is provided with a diameter substantially the same as the operation wire 20, but the portion is provided with some allowance considering insertability of the operation wire 20.

In addition, as shown in Fig. 4, a projection portion 31 is formed at each of the bending rings 10. The projection portion 31 is a portion formed by denting the bending ring 10 inward by a predetermined height and is provided at the distal end side and the base end side around the cut and drawn portion 30 and in the vertical direction respectively. Thus, the projection portions 31 are provided at least at four spots on each of the binding rings 10. The projection portion 31 is formed so that it makes a smooth shape, that is, the entire projection portion 31 makes a curved line. The bending ring 10 is created by pressing and the like, and the projection portion 31 can be formed by carrying out machining so that a dent having a smooth pattern is formed at the position of the projection portion 31 at pressing of the bending ring 10. In this example, the projection portion 31 is formed by denting the bending ring 10 inward by a predetermined height, but another member corresponding to the projection portion 31 may be provided. Furthermore, the projection portion 31 is formed at front and rear positions in the axial direction of the cut and drawn portion 30, that is, at the same positions in the circumferential direction. At this time, since the operation of the operation wire 20 in the circumferential direction is regulated, the projection portion 31 and the operation wire 20 are located at the same spot in the circumferential direction.

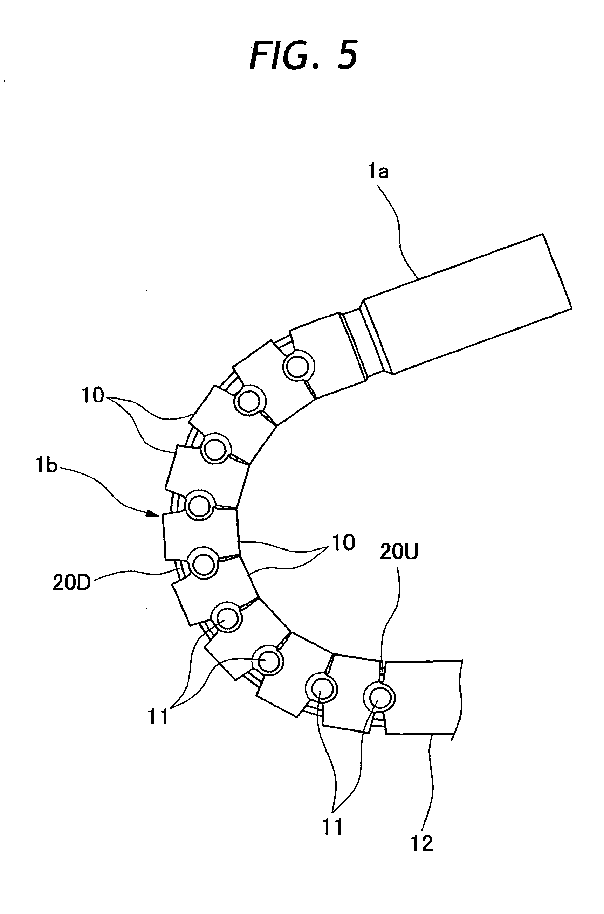

Fig. 5 illustrates the maximum bent state of the bending tube portion 1b. Supposing that the operation wire on the side pulled toward the base end side is 20U and the operation wire on the side pulled out from the base end side is 20D, in the maximum bent state, the operation wire 20U loses the interval G formed between front and rear rings of all the bending rings 10 provided on the front and rear positions and the interval doubles (that is, 2G) on the operation wire 20D side, and thus, the bending tube portion 1b is bent into an arc shape. The operation wires 20U and 20D have the arc shape at this time, but the radius of curvature of the arc is smaller for the operation wire 20U. Therefore, a pressing force toward the inner circumferential face of the bending ring 10 acts on the operation wire 20U and particularly in the maximum bent state, the pressing force applied to the operation wire 20U is large.

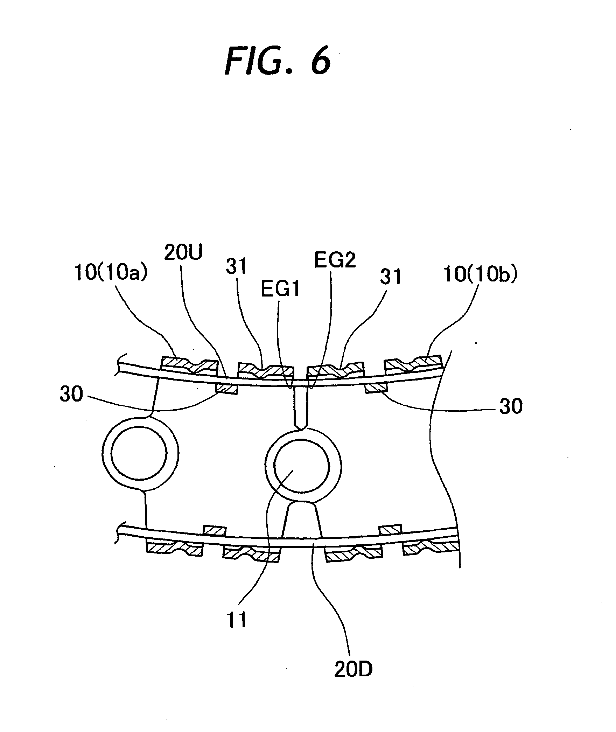

Fig. 6 shows a coupling portion between the front and rear bending rings 10 (supposing that one bending ring is 10a and the other bending ring is 10b) when the bending tube portion 1b is bent to some degree. At both end portions of the bending ring 10, an edge (supposing that an edge of the bending ring 10a is EG1 and an edge of the bending ring 10b is EG2) is formed by a process of cutting out. When the bending tube portion 1b is bent as mentioned above, since the pressing force acts on the operation wire 20U toward the inner circumferential face of the bending ring 10, the operation wire 20U is to move toward the edge EG1 and the edge EG2. At this time, if the pushing / pulling operation is applied while the operation wire 20U is in contact with the edges EG1, EG2, the operation wire 20U is rubbed by the edges EG1, EG2 and damaged.

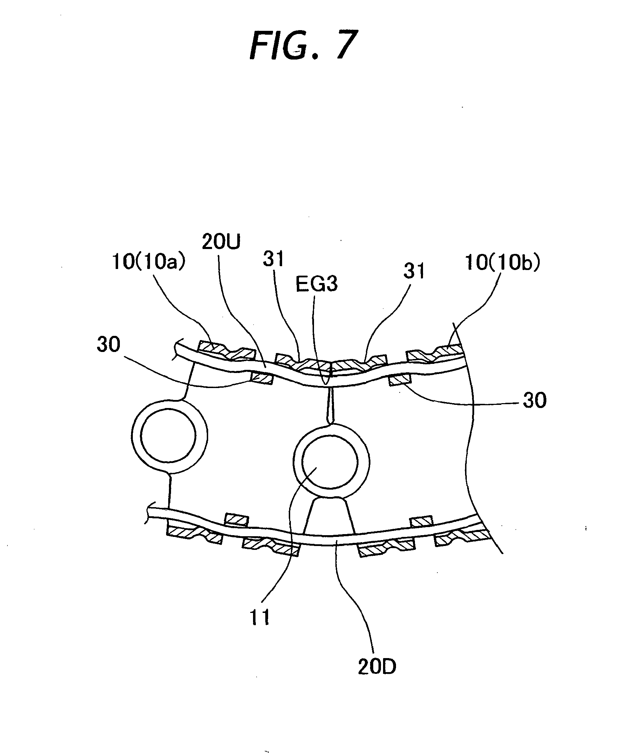

Fig. 7 also shows a coupling portion of the front and rear bending ring 10 when the bending tube portion 1b is in the maximum bent state, but in this state, a new edge EG3 is formed by the edge EG1 of the bending ring 10a and the edge EG2 of the bending ring 10b. The edge EG3 directs a sharply-angled top in a direction opposite to the pressing direction of the operation wire 20U, and a degree of damage to the operation wire 20U is larger than the case in Fig. 6. Since the bending tube portion 1b can no longer be bent while it is in the maximum bent state, rubbing is negligibly caused by the pushing / pulling operation of the operation wire 20U, however, a slight movement in the front and rear direction is possible. If the sharply-angled top rubs the operation wire 20U at this time, the abrasion degree of the operation wire 20U increases considerably. Also, even without the movement in the front and rear direction, if the operation wire 20U is repeatedly pressed onto the edge EG3 at the maximum bending, the damage to the operation wire 20U becomes more serious due to the pressing.

In the present invention, as shown in Figs. 6 and 7, the inward projection portion 31 is formed in the bending ring 10. The projection portion 31 is provided in a direction opposite the direction in which the operation wire 20U is pressed when the bending tube portion 1b is bent. Also, the projection portion 31 is provided for each of the bending rings 10, and even if the operation wire 20U is moved toward the inner circumferential face of the bending ring 20, the operation wire 20U is no longer moved from a position brought into contact with both the projection portions 31 by means of the projection portion 31 of the bending ring 10a and the projection portion 31 of the bending ring 10b. The edges EG1, EG2, EG3 (collectively called edge EG) are formed at an intermediate position between both the projection portions 31, and since the operation of the operation wire 20U is regulated by the front and rear projection portions 31, the operation wire 20U does not contact the edge EG.

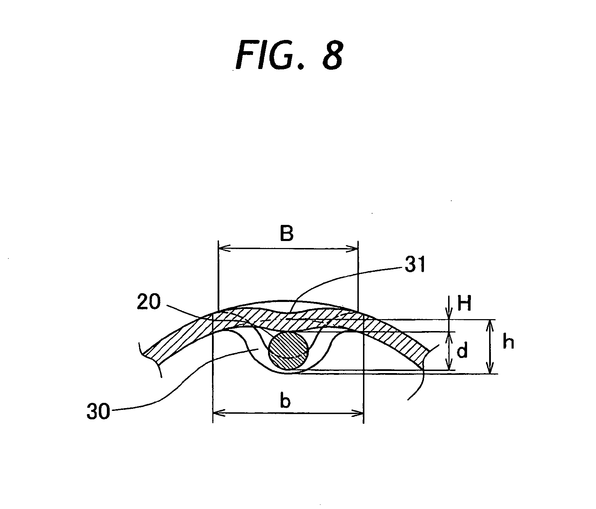

Here, though the operation in the circumferential direction of the operation wire 20U is regulated by the cut and drawn portion 30, the operation wire 20 has room to be operated in the circumferential direction freely to some degree between the cut and drawn portions 30 of the front and rear bending rings 10. At this time, if the width of the projection portion 31 in the circumferential direction is small, the operation wire 20 is removed from the projection portion 31 and might contact the edge EG. Thus, as shown in Fig. 8, some width is given to the projection portion 31 in the circumferential direction. As a result, the operation wire 20 can be brought into contact with the projection portion 31 all the time. In addition, by forming a portion of the projection portion 31 projecting the most inward in a flat state, even if the operation wire 20U is slightly operated in the circumferential direction, the wire will not drop from the projection portion 31.

A height and a width of the projection portion 31 will be described. The projection portion 31 is provided in order to avoid rubbing between the operation wire 20U and the edge EG of the bending ring 10, and ideally, the height is preferably such that the operation wire 20U and the edge EG are not in contact with each other even if the bending tube portion 1b is brought into the maximum bent state. However, even if the operation wire 20U and the edge EG are in slight contact with each other, provision of the projection portion 31 can alleviate the pressing force toward the edge EG applied to the operation wire 20U, and the operation wire 20U can be protected. From this point of view, when the height of the projection portion 31 is H, a relation of H > 0 is required as a condition.

On the other hand, if the projection portion 31 is set high, the projection portion 31 protrudes largely into the inner circumference side of the bending tube portion 1b, by which the capacity for the contents is reduced. Thus, it is not preferably to set the height of the projection portion 31 higher than necessary. Here, the cut and drawn portion 30 is already provided at the bending ring 10, and if the height is set up to the position of the cut and drawn portion 30 which has been originally provided, setting the projection portion 31 high does not have a negative impact. Thus, the height H of the projection portion 31 is set so that "H ≤ h - d" is satisfied when the diameter of the operation wire 20 is d, and the height of the cut and drawn portion 30 is h, as shown in Fig. 8. As a result, the influence of the reduced capacity by the projection portion 31 can be eliminated. If H is set high under this condition, the capacity of the flexible tube portion 1c is not reduced, and damage to the operation wire 20U can be avoided.

Subsequently, the width of the projection portion 31 will be described. As mentioned above, some width is given to the projection portion 31 in the circumferential direction, but if a width larger than necessary is given to the projection portion 31, the capacity of the flexible tube portion 1c is reduced. Here, the cut and drawn portion 30 is already provided at the bending ring 10, and if the width is within a range of the width of the cut and drawn portion 30 which has been originally provided, giving a width to the projection portion 31 does not matter. Thus, the width of the projection portion is set so that "B ≤ b" is satisfied when the width of the projection portion 31 is B and the width of the cut and drawn portion 30 is b in the circumferential direction. As a result, the influence of the reduced capacity can be eliminated. Then, if B is set to be larger under this condition, the capacity of the flexible tube portion 1c is not reduced and the operation wire 20 can be prevented from being removed.

However, the above conditions of "H ≤ h - d" related to the height H of the projection portion 31 and "B ≤ b" related to the width B are set with the purpose of eliminating the influence caused by reduced capacity, and if some influence can be allowed, the above condition expressions do not have to be met.

Furthermore, by forming the shape of the projection portion 31 in a recess shape, the removal of the operation wire 20U can be avoided while the width B for the projection portion 31 is set to be small. By forming the projection portion 31 in the recess shape, the operation wire 20U is fitted in the dent portion, by which a function to regulate operation in the circumferential direction can be given to the projection portion 31. Therefore, the removal of the operation wire 20U can be prevented without significantly increasing the width of the projection portion 31.

Subsequently, a position of the projection portion 31 in the axial direction will be described. As shown in Figs. 6 and 7, by providing the projection portion 31 at the bending ring 10, the operation wire 20 is prevented from contacting the edge EG of the bending ring 10. In this regard, the projection portion 31 is preferably formed in the neighborhood of the end portion of the bending ring 10 as much as possible. The projection portion 31 is provided in order to protect the operation wire 20 from the edge EG, and by providing the projection portion 31 at the position close to a spot where the edge EG is formed as much as possible, the operation wire 20 can be prevented from being touched by the projection portion 31 more surely.

However, if the vicinity of the end portion of the bending ring 10 is worked in order to form the projection portion 31, durability and the like of the vicinity of the edge EG is lowered. Particularly, if the bending tube portion 1b is brought into the maximum bent state, since the end portions of the bending rings 10 are brought into contact with each other. Since the durability in the vicinity of the edge EG of the bending ring 10 is lowered, the contact might cause deformation or even damage. Then, the position of the projection portion 31 in the axial direction is preferably located at a position close to the end portion as much as possible and so as not to lower the durability of the vicinity of the edge such as an intermediate position between the cut and drawn portion 30 and the end portion, for example.

In the above, the bending tube portion 1b capable of being bent in the two vertical directions using the two operation wires 20, 20 has been described, but four operation wires may be provided. The four operation wires are arranged with an interval of 90 degrees in the circumferential direction of the bending tube portion 1b and are capable of being bent in four vertical and horizontal directions. The projection portion 31 in this case may be provided corresponding to each of the operation wires but is preferably formed in the circumferential direction of the bending ring 10, that is, over the entire circumference of 360 degrees.

## Claims

1. An endoscope insertion portion (1) comprising:
a distal-end rigid portion (1a) having at least an illumination portion (4) and an observation portion (5);
a bending tube portion (1b) coupled to the distal-end rigid portion (1a) and to which a plurality of bending rings (10) are coupled by a pivot attachment portion (11); and
a flexible tube portion (1c) coupled to the bending tube portion (1b) and a main body operation portion (2), wherein
said bending ring (10) is provided with an insertion path through which an operation wire (20) for bending said bending tube portion (1b) by a remote control from said main body operation portion (2) is inserted; **characterised by**
an inward projection portion (31) provided in the front and rear position in the axial direction of the insertion path.

2. The endoscope insertion portion (1) according to Claim 1, wherein
said projection portion (31) is given an extra width in the circumferential direction of said bending ring.

3. The endoscope insertion portion (1) according to Claim 1 or 2, wherein
said projection portion (31) is formed in a recess state.

4. The endoscope insertion portion (1) according to any of Claims 1 to 3, wherein
a height of said projection portion (31) is set so that "H ≤ h - d" is satisfied when the height of said projection portion (31) is H, the height of said insertion path is h, and a diameter of said operation wire (20) is d.

## Patentansprüche

1. Endoskopeinführteil (1), umfassend:
einen starren Distalendeteil (1a) mit mindestens einem Beleuchtungsabschnitt (4) und einem Beobachtungsabschnitt (5);
einen an den starren Distalendeteil (1a) gekoppelten Biegerohrteil (1b), an den über einen Schwenkbefestigungsteil (11) mehrere Biegeringe (10) gekoppelt sind; und
einen flexiblen Rohrteil (1c), der an den Biegerohrteil (1b) und an einen Hauptkörperbetätigungsteil (2) gekoppelt ist, wobei
der Biegering (10) mit einem Einführweg ausgestattet ist, durch den ein Betätigungsdraht (20) zum Biegen des Biegerohrteils (1b) mit Hilfe einer Fernsteuerung seitens des Hauptkörperbetätigungsteils (2) eingeführt ist; **gekennzeichnet durch**
einen an der in axialer Richtung vorderen und hinteren Stelle des Einführwegs vorgesehenen nach innen gerichteten Vorsprung (31).

2. Endoskopeinführteil (1) nach Anspruch 1, bei dem
der Vorsprung (31) in Umfangsrichtung des Biegerings eine Extrabreite aufweist.

3. Endoskopeinführteil (1) nach Anspruch 1 oder 2, bei dem
der Vorsprung (31) in einem Vertiefungszustand ausgebildet ist.

4. Endoskopeinführteil (1) nach einem der Ansprüche 1 bis 3, bei dem
eine Höhe des Vorsprungs (31) derart eingestellt ist, dass "H ≤ h - d" erfüllt ist, wenn die Höhe des Vorsprungs (31) H beträgt, die Höhe des Einführwegs h beträgt und der Durchmesser des Betätigungsdrahts (20) d beträgt.

## Revendications

1. Partie d'insertion d'endoscope (1), comprenant :
une partie rigide d'extrémité distale (1a), présentant au moins une partie d'éclairement (4) et une partie d'observation (5) ;
une partie de tube à flexion (1b) couplée à la partie rigide d'extrémité distale (1a) et à laquelle une pluralité d'anneaux à flexion (10) sont couplés par une partie de fixation à pivot (11), et
une partie de tube flexible (1c) couplée à la partie de tube à flexion (1b) et à une partie de manoeuvre de corps principale (2), dans laquelle
ledit anneau à flexion (10) est doté d'un trajet d'insertion à travers lequel est inséré un fil de manoeuvre (20) destiné à la flexion de ladite partie de tube à flexion (1b) par une commande à distance à partir de ladite partie de manoeuvre de corps principale (2), **caractérisée par**
une partie faisant saillie vers l'intérieur (31) prévue dans les positions avant et arrière dans la direction axiale du trajet d'insertion.

2. Partie d'insertion d'endoscope (1) selon la revendication 1, dans laquelle
ladite partie faisant saillie (31) reçoit une largeur supplémentaire dans la direction circonférentielle dudit anneau à flexion.

3. Partie d'insertion d'endoscope (1) selon la revendication 1 ou 2, dans laquelle
ladite partie faisant saillie (31) est formée dans un état en retrait.

4. Partie d'insertion d'endoscope (1) selon l'une quelconque des revendications 1 à 3, dans laquelle
une hauteur de ladite partie faisant saillie (31) est fixée de telle sorte que « H≤h-d » est satisfaite lorsque la hauteur de ladite partie faisant saillie (31) est H, la hauteur dudit trajet d'insertion est h, et un diamètre dudit fil de manoeuvre (20) est d.
